# EUROPEAN PATENT SPECIFICATION

(11) **EP 1 545 671 B1**
(45) Date of publication and mention of the grant of the patent: **04.07.2012**
(21) Application number: 03797306.2
(22) Date of filing: 15.09.2003
(51) Int. Cl.: A61M 15/00

(54) **METHOD FOR LOADING A MEDICAMENT DISPENSER WITH A MEDICAMENT CARRIER**
VERFAHREN ZUM LADEN EINES MEDIKAMENTENSPENDERS MIT EINEM ARZNEIMITTELTRÄGER
PROCEDE DE CHARGEMENT D'UN DISTRIBUTEUR DE MEDICAMENT AVEC UN SUPPORT DE MEDICAMENT

(30) Priority: 17.09.2002 GB 0221493
(43) Date of publication of application: 29.06.2005
(73) Proprietor: GLAXO GROUP LIMITED, Greenford, Middlesex UB6 0NN (GB)
(72) Inventor: ANDERSON, Gregor John McLennan, Ware, Hertfordshire SG12 0DP (GB); DUFFIELD, Howard Peter, Ware, Hertfordshire SG12 0DP (GB); THOMAS, Tudor Hugh, Ware, Hertfordshire SG12 0DP (GB)
(74) Representative: Rice, Jason Neale
(86) International application number: PCT/EP2003/010347
(87) International publication number: WO 2004/026378

(56) References cited:
- EP-A- 0 467 172
- EP-A- 1 132 104
- WO-A-02/36189
- WO-A-02/078594
- WO-A-03/061743
- WO-A-03/061744
- US-A- 4 090 642
- US-A1- 2002 053 344

## Description

### Technical field

The present invention relates to a method for loading a medicament dispenser with a medicament carrier. The invention particularly relates to method for loading a multi-unit dose medicament dispenser with a medicament carrier in elongate strip form.

### Background to the invention

The use of medicament dispensers in the administration of medicaments, for example in bronchodilation therapy is well known. Such devices generally comprise a body or housing within which a medicament carrier is located. Known inhalation devices include those in which the medicament carrier is an elongate blister strip containing a number of discrete doses of powdered medicament. In use, the elongate strip is housed within the dispenser in such a way that the strip may be transported through the dispenser in indexed fashion to enable accessing of the discrete doses of medicament carried thereby. Such devices usually contain a mechanism of individually accessing the doses, usually comprising either piercing means or means to peel a lid sheet away from a base sheet. The powdered medicament can then be accessed and inhaled. Such a mechanism may also be used for dispensing medicament in tablet form wherein peeling away the lid sheet from the base sheet reveals a tablet for removal and subsequent consumption.

The efficient loading of an elongate strip form medicament carrier into a medicament dispenser presents numerous problems from a manufacturing assembly standpoint, particularly where such assembly is to be carried out on a high-speed production line. Particular problems include reducing the number and complexity of assembly steps required to introduce the strip into the dispenser; ensuring the correct location of the strip within the dispenser; and ensuring the correct tensioning and transport characteristics of the strip within the dispenser to enable effective indexing and access.

One known method of loading an elongate strip medicament carrier requires the use of either a two-part shell or hinged clamshell form medicament dispenser. Initially, the shell is open to enable the direct placement of the medicament carrier strip therein. At least one end of the carrier strip is then anchored to a drive element (e.g. a drive wheel) within the dispenser which enables the drivable movement thereof. Once the strip is appropriately located, anchored and optionally tensioned, the shell is then closed up to provide the loaded dispenser. Prior to use, it may also be necessary to prime the dispenser such that the medicament carrier strip is transported within the dispenser to an initial dose access position.

The Applicant has realized that it can be beneficial to pre-coil the medicament carrier prior to loading it into the housing of the medicament dispenser. From the document WO 03/061743 there is known coiled medicament carriers used in dry powder inhalers. Pre-coiling methods are therefore disclosed herein, including static and dynamic coiling methods. The pre-coiled medicament carrier may be loaded into the housing as is, or it may be associated with some kind of retaining means designed to retain its coiled form. Suitable retaining means are therefore also disclosed.

### Summary of the invention

According to the invention there is provided a method of coiling a medicament carrier as set forth in claim 1.

The method herein is suitable for loading a medicament dispenser with one or more medicament carriers having multiple distinct medicament doses carried thereby.

In one aspect, the medicament dispenser has unitary form and the housing is integral therewith. In another aspect, the medicament dispenser is configured to receive a refill cassette and the housing forms part of that refill cassette.

The medicament dispenser is shaped to receive one or more elongate strip form medicament carriers. Suitable elongate form medicaments carriers are in the form of a strip or tape. The term medicament carrier is used to define any suitable carrier. In a preferred aspect, the carrier has a blister pack form, particularly a blister strip having multiple distinct blister portions provided along its length, but it could also, for example, comprise a carrier onto which medicament has been applied by any suitable process including printing, painting and vacuum occlusion. The medicament carrier has multiple distinct (i.e. separate) medicament doses carried thereby.

In one aspect, the medicament carrier comprises a blister pack in laminate form. Suitably, the laminate comprises material selected from the group consisting of metal foil, organic polymeric material and paper. Suitable metal foils include aluminium or tin foil having a thickness of from 5 to 100 µm, preferably from 10 to 50µm, such as 20 to 30µm. Suitable organic polymeric materials include polyethylene, polypropylene, polyvinyl chloride and polyethylene terephthalate.

Access to the medicament dose portions comprised within the pockets of the elongate strip form carrier is by any suitable access means including tearing, piercing or peeling apart the relevant pockets.

One suitable blister pack form medicament carrier comprises a peelable blister strip. Suitably, the peelable blister strip comprises a base sheet in which blisters are formed to define pockets therein for containing distinct medicament dose portions and a lid sheet which is hermetically sealed to the base sheet except in the region of the blisters in such a manner that the lid sheet and the base sheet can be peeled apart. The base and lid sheets are typically sealed to one another over their whole width except for the forward end portions where they are typically not sealed to one another at all. Thus, separate base and lid sheet forward end portions are presented at the end of the strip. The respective base and lid sheets are peelably separable from each other to (e.g. separately) release the contents of each pocket.

Suitably, the lid sheet comprises at least the following successive layers: (a) paper; adhesively bonded to (b) polyester; adhesively bonded to (c) aluminium foil; that is coated with a heat seal lacquer for bonding to the base sheet. The thickness of each layer may be selected according to the desired properties but is typically of the order of from 5 to 200 micron, particularly from 10 to 50 micron.

Suitably, the base sheet comprises at least the following successive layers: (a) oriented polyamide (OPA); adhesively bonded to (b) aluminium foil; adhesively bonded to (c) a third layer comprising a polymeric material (e.g. polyvinyl chloride).

Various known techniques can be employed to join the lid and base sheet and hence to seal the blisters of the peelable blister strip. Such methods include adhesive bonding, hot metal bonding, hot metal welding, radio frequency welding, laser welding, ultrasonic welding and hot bar sealing. The lid sheet and base sheet of the peelable blister strip are particularly sealable by 'cold form' sealing methods, which are conducted at lower temperatures than conventional heat sealing methods. Such 'cold form' sealing methods are of particular utility where the medicament or medicament formulation for containment within the blister is heat sensitive (e.g. degrades or denatures on heating). Suitable 'cold form' sealing methods are conducted at a temperature in the range of 150-250°C, more preferably, 210-240°C.

The medicament dispenser may have an internal mechanism for dispensing the distinct medicament doses carried by the medicament carrier for administration to the patient (e.g. by inhalation). Suitably, the mechanism comprises,
a) receiving means for receiving the medicament carrier,
b) release means for releasing a distinct medicament dose from the medicament carrier on receipt thereof by said receiving means;
c) an outlet, positioned to be in communication with the medicament dose releasable by said release means; and
d) indexing means for individually indexing the distinct medicament doses of the medicament carrier.

Where the medicament dispenser is arranged to receive plural elongate form medicament carriers variations of the above mechanism are envisaged, in which each medicament carrier is suitably transported within the dispenser.

The internal mechanism comprises receiving means (e.g. a receiving station) for receiving the or each medicament carrier.

The mechanism further comprises release means for releasing a distinct medicament dose from the or each medicament carrier on its receipt by the receiving station. The release means can have any suitable form. Where the elongate carrier is in the form of a blister strip, the release means may for example, be a means to rupture or otherwise access the blister. In a particular preferred aspect, where the blister strip is peelably accessible, the release means comprises means for peeling apart the blister strip.

An outlet is positioned to be in communication with the distinct medicament doses releasable by said release means. The outlet may have any suitable form. In one aspect, it has the form of a mouthpiece and in another; it has the form of a nozzle for insertion into the nasal cavity of a patient.

The outlet is preferably a single outlet, which communicates with the distinct medicament dose releasable by said release means via a common air channelling means (e.g. formed as an air-pipe or common manifold). The patient may therefore breathe in through a single outlet, and that breath be transferred through the common channelling means to the released medicament dose, thereby enabling its inhalation. Baffles or other mechanical aids to break up released medicament powder may be incorporated. Venturi channelling of the air flow is also envisaged in embodiments. Helical form channel are envisaged.

The internal mechanism also comprises indexing means for individually indexing the distinct medicament doses of the or each medicament carrier. Said indexing typically happens in sequential fashion, for example accessing dose portions sequentially arranged along the length of the elongate carrier.

The method of the present invention provides a method of pre-coiling an elongate form medicament carrier prior to loading it into the medicament dispenser.

The pre-coiling method may be carried out prior to fixing of the medicament carrier to a leader and its loading Into the housing. Alternatively, the method of pre-coiling is carried out prior to loading of the medicament carrier into the housing without any use of leaders to guide the medicament carrier into the housing.

The spindle may be of diameter from 4 to 8mm. The spindle is both rotatable and movable laterally.

When the spindle is rotated and the strip is also moved laterally, overall, the coiled strip thereby moves laterally as it forms.

The pre-coiled medicament carrier may be loaded as is, or it may be associated with some kind of retaining means designed to retain its coiled form. The retaining means may comprise a simple dip or it may take the form of a cassette housing or part-housing that is shaped for engagement with the housing for the medicament dispenser. In one particular aspect, the retaining means takes the form of a closure for a hatch that is shaped for engagement (e.g. snap-fit) with an access hatch (or window) of the housing for the medicament carrier.

In a preferred aspect, the medicament carrier comprises a peelable blister strip having a plurality of pockets for containing medicament wherein said pockets are spaced along the length of and defined between two peelable sheets secured to each other. The respective peelable sheets are generally in the form of a base sheet and a lid sheet of a pocket. In this aspect, the release means comprises peeling means for peeling apart a base sheet and lid sheet to open a pocket Suitably, the peeling means includes lid-driving means for pulling apart a lid sheet and a base sheet of a pocket that has been received at the opening station.

### Brief Description of the Drawings

The invention will now be described with reference to the accompanying drawings in which:
Figure 1 shows a perspective view of a medicament carrier suitable for use in accord with the present invention;
Figure 2 shows in plan view a base unit of a medicament dispenser including an internal mechanism;
Figure 3 shows a perspective view of a medicament dispenser, in the form of a holder/body and a refill cassette with the cassette removed from the holder/body;
Figures 4a to 4f show in plan view sequential steps involved in the pre-loading of a leader strip portion and loading of an elongate strip form medicament carrier;
Figures 5a and 5b each show a perspective view of a medicament cassette having a hinged access portion;
Figure 6 shows a plan view of a spool for receipt of the end of a medicament carrier;
Figures 7a and 7b respectively show in perspective view a medicament dispenser having a cut-away access portion and a coiled medicament carrier for receipt thereby;
Figures 8a to 8c respectively show methods of coiling a medicament carrier in accordance with the invention; and
Figures 9a to 9d show in plan view, and Figure 9e in perspective view, sequential steps involved in the pre-coiling of an elongate strip form medicament carrier and its loading into a medicament dispenser housing in accord with the invention.

### Detailed Description of the Drawings

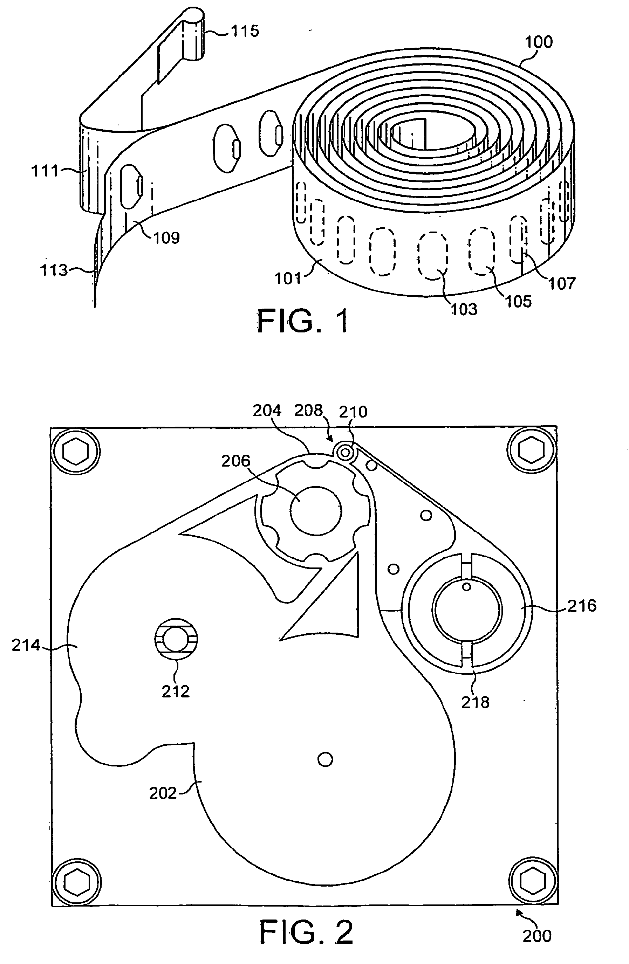

Figure 1 shows a medicament carrier 100 for use in accord with the present invention. The medicament carrier comprises a flexible strip 101 defining a plurality of pockets 103, 105, 107 each of which would contain a portion of a dose of medicament which can be inhaled, in the form of powder.

The strip comprises a base sheet 109 in which blisters are formed to define the pockets 103, 105, 107 and a lid sheet 111 which is hermetically sealed to the base sheet except in the region of the blisters in such a manner that the lid sheet 111 and the base sheet 109 can be peeled apart. The sheets 109. 111 are sealed to one another over their whole width except for the leading end portions 113, 115 where they are preferably not sealed to one another at all.

The lid 111 and base 109 sheets are each formed of a plastics/aluminium laminate and are suitably adhered to one another by heat sealing. The lid sheet 111 comprises at least the following successive layers: (a) paper; adhesively bonded to (b) polyester; adhesively bonded to (c) aluminium foil; that is coated with a heat seal lacquer for bonding to the base sheet. The base sheet 109 comprises at least the following successive layers: (a) oriented polyamide (OPA); adhesively bonded to (b) aluminium foil; adhesively bonded to (c) a third layer comprising a polymeric material (e.g. polyvinyl chloride).

The strip 101 is shown as having elongate pockets 103, 105, and 107 that run transversely with respect to the length of the strip 101. This is convenient in that it enables a large number of pockets 103, 105, 107 to be provided in a given strip 109 length. The strip 101 may, for example, be provided with thirty, sixty or one hundred pockets but it will be understood that the strip 101 may have any suitable number of pockets.

Figure 2 illustrates a base unit 200 of a medicament dispenser suitable for use in the present invention. A blister strip (not shown for clarity) is positioned in chamber 202 of the base unit 200. The blister strip is pre-fed through a guide member 204 within the manifold component and engaged in a six-pocket index wheel 206. The first pocket of the blister strip is positioned one pocket away from the opening station 208. The lid foil and base foil are separable about a beak 210. The resulting empty base foil is coiled about a base take-up spindle 212 in the base take-up chamber 214. The used lid foil is fed over the beak 210 and coiled about a lid take-up spindle 216 in the lid take-up chamber 218.

The dispenser is actuated by pressing a button on the side of the dispenser (not shown) to index the internal mechanism by one pocket of medicament Initially, the gearing between the index wheel 206 and the lid take-up foil spindle 216 is one-to-one. However, as the lid take up spindle 216 winds on more foil, its effective winding diameter increase. An increase in diameter would cause the lid take-up spindle 216 to pull more strip than the index wheel 206 releases.

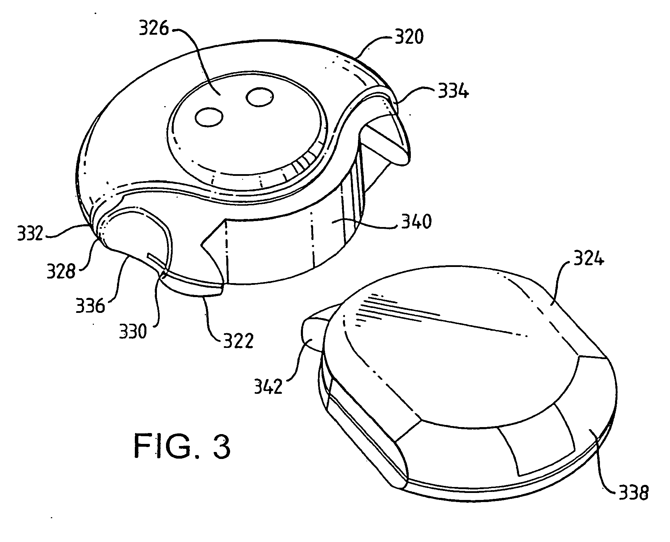

Figure 3 shows a prior art medicament dispenser of a form suitable for use in the present invention, comprising a body 320, a holder 322, refill cassette 324 and electronic display 326. The holder 322 is shaped to fit snugly inside body 320 and is fixed to a point on the body (not shown) about which it rotates. Stops 328, 330 protrude from the holder 322 and prevent the holder 322 from rotating more than about 180° relative to the body 320. The stops 328, 330 also provide two defined positions of the holder 322 within the body 320. One position is defined by stop 328 meeting with body edge 332 and the other position defined by stop 330 meeting with body edge 334 when the holder has been rotated relative to the body. The area between stops 328 and 330 is shaped to form a thumb or finger grip 336 for the user of the device. The holder 322 forms a shell into which the refill cassette 304 snugly fits.

The refill cassette 324 comprises a shell containing the plural medicaments carrier (not shown) and a mechanism for opening the carriers (not shown) for the medicament to be accessed. The refill cassette 324 has a raised portion 338 at one end on both sides along its width so that this part of the refill cassette 324 is at least the same depth as the interior part 340 of the holder 322 which receives the refill cassette 324. This allows the position of the cassette 324 within the holder 322 to be fixed such that the ridge 338 protrudes from the holder 322 but the rest of the cassette 324 is contained within the holder 322.

The refill cassette 324 also has a mouthpiece (not shown) and an actuating push button 342 for actuating the device to index the medicament carrier within the cassette 324.

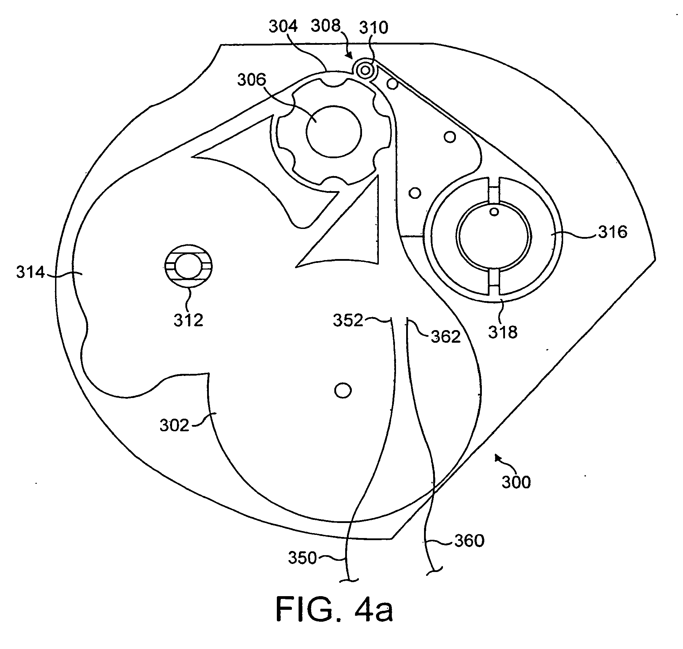

Figures 4a to 4f illustrate successive steps involved in loading a base unit housing 300 of a medicament dispenser with a medicament carrier. The base unit housing 300 may either be comprised as an integral medicament dispenser as such, or as a refill cassette for a medicament dispenser. The features of the housing 300 are described first followed by the features of the method.

The internal mechanism of the medicament dispenser of Figures 4a to 4f is based on that of the dispenser of Figure 2. A base unit 300 includes chamber 302 for receipt of an elongate medicament carrier in the form of a blister strip. The received blister strip is pre-fed through a guide member 304 within the manifold component and engaged in a six-pocket index wheel 306. Opening of pockets of the blister strip is configured to occur at the opening station 308. In dispensing use, the lid foil and base foil are separable about a beak 310. The resulting empty base foil is coiled about a base take-up spindle 312 in the base take-up chamber 314. The used lid foil is fed over the beak 310 and coiled about a lid take-up spindle 316 in the lid take-up chamber 318.

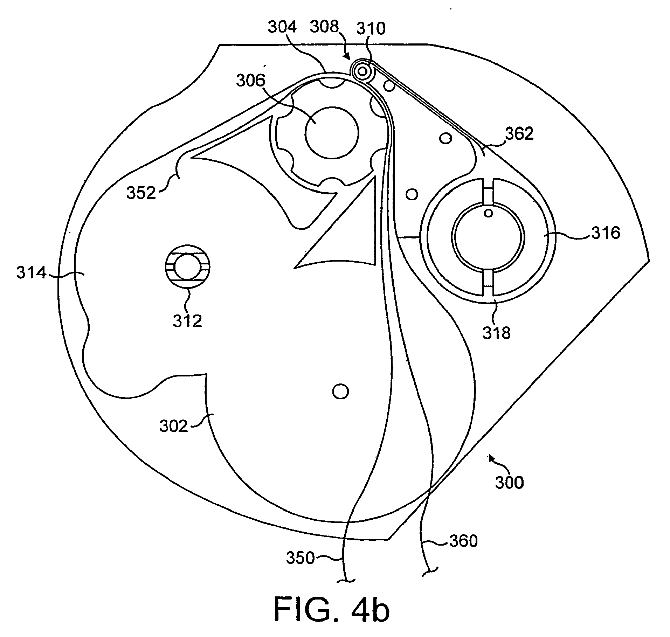

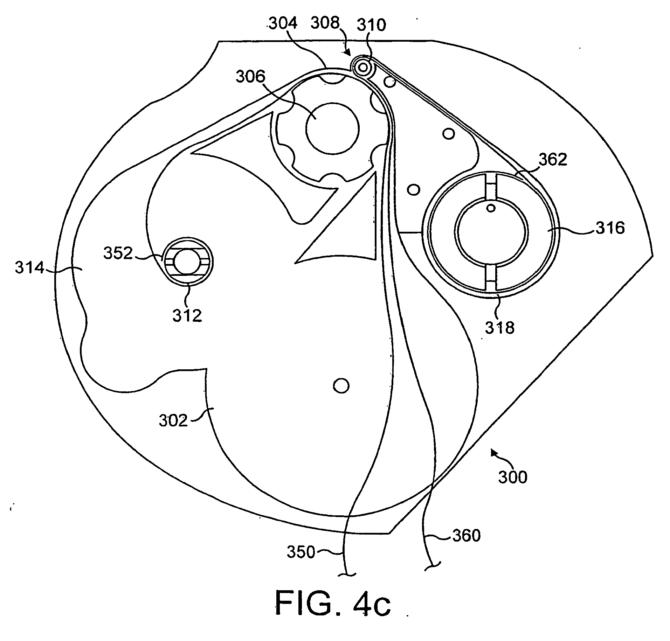

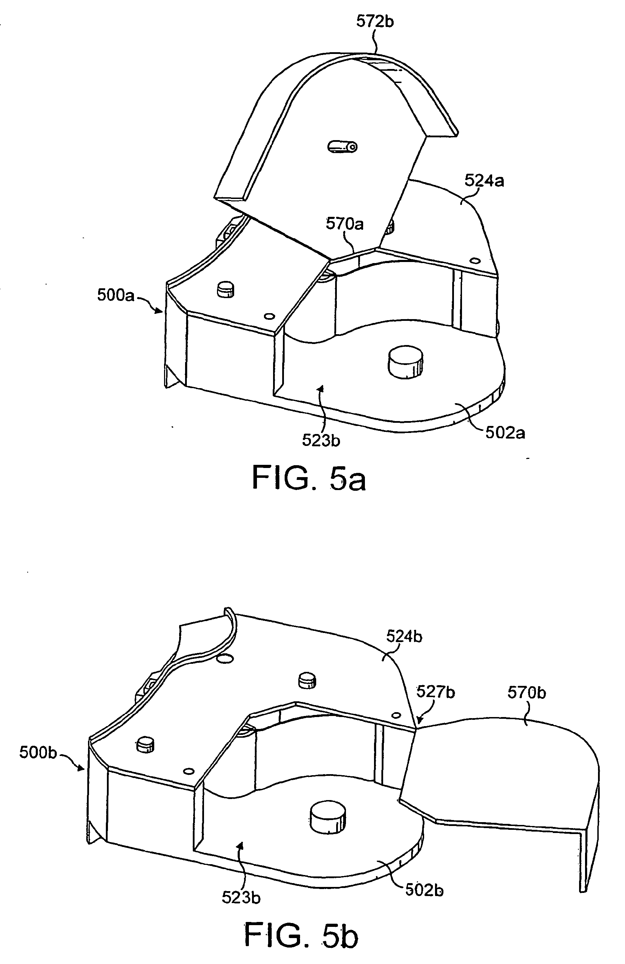

Turning to now the loading method, in Figure 4a two leader strips 350, 360, each comprising a plain tape formed of plastic polymer, paper, metal, fabric or a laminate are introduced to the base unit 300 via the chamber 302. Access to the chamber may be provided in a variety of ways, particularly as illustrated in Figures 5a and 5b. It may be seen that the leading ends 352, 362 of each respective leader strip are directed towards the opening mechanism 304, 306, 308 of the dispenser 300. In Figure 4b, those leading ends 352, 362 have been received within different parts of the opening mechanism 304, 306, 308. In more detail, the first leader strip 350 is directed around the index wheel 306 and its leading end 352 guided towards the base take-up chamber 314. The second leader strip 360 is directed around the beak 310 and its leading end 362 guided towards the lid take-up chamber 318. In Figure 4c, the respective leading ends 352, 362 of the leader strips 350, 360 are secured to the base take-up spindle 316 and lid take-up spindle 318. The leader strips 350, 360 may now thus, be transported through the dispenser 300 by respective rotation of the relevant take-up spindles 316, 318 in much the same way that a medicament carrier would be transported through the dispenser in use (e.g. as described previously in relation to the dispenser of Figure 2).

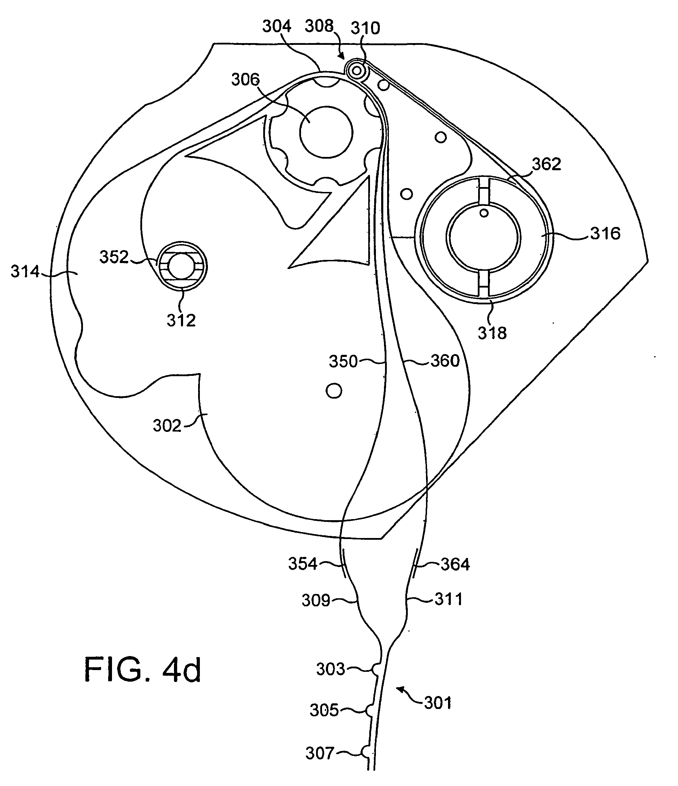

In manufacturing aspects, the leader strip threaded 'product' of the method steps shown in Figures 4a to 4c may be regarded as a sub-assembly. In one aspect herein, this sub-assembly may be manufactured at a site that is distinct, and potentially geographically distant, from the site at which the subsequent steps of Figures 4d to 4f (described below) are conducted.

It will have been appreciated from the above that the first leader strip 350 of the sub-assembly is designed to function as a leader for a base sheet, and the second leader strip 360 as a leader for a lid sheet, wherein the medicament carrier has the general form of that shown in Figure 1.

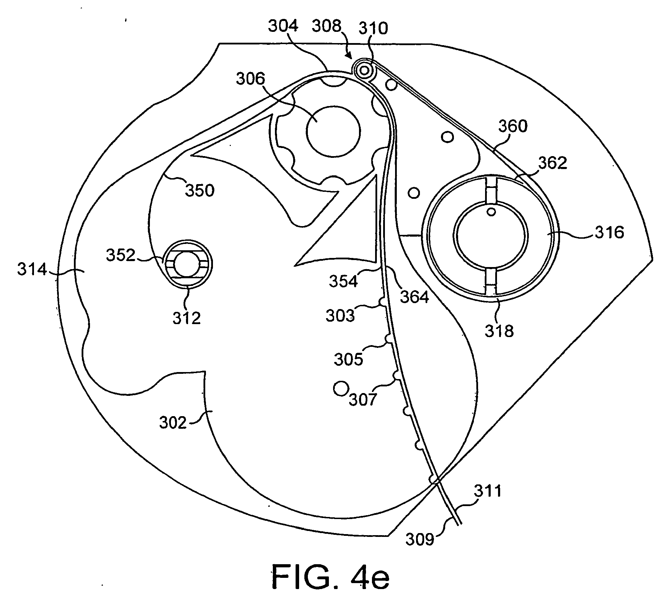

In Figure 4d, the trailing end 354 of the first leader strip 350 is fixed to the base sheet 309 and the trailing end 364 of the second leader strip 360 is fixed to the lid sheet 311 of an elongate form blister strip 301. The fixing may be achieved by any suitable means including adhesive and welding methods. It will now be appreciated that the respective leader strips 350, 360 may be employed to 'lead' the blister strip into the internal mechanism 304, 306, 308 of the dispenser. Thus, in Figure 4e the leader strips 350, 360 have been advanced by rotation of the respective take-up spindles 316, 318 and the blister strip 301 thereby drawn into the dispenser 300.

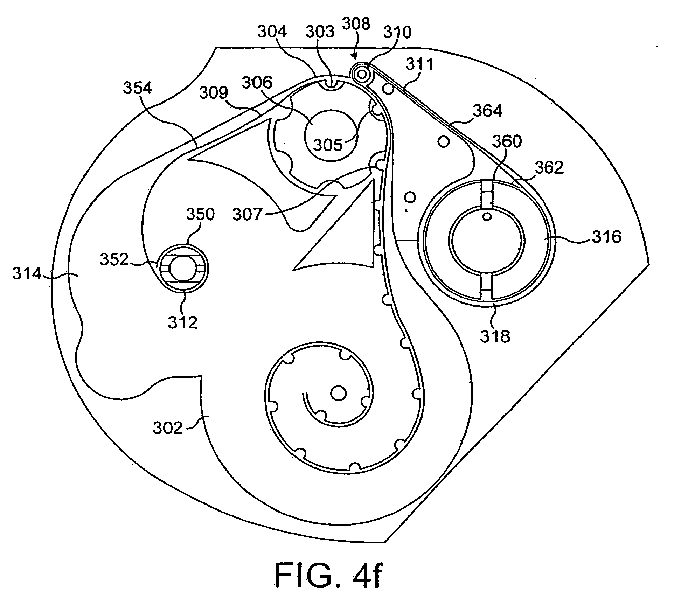

In Figure 4f, the blister strip has been further drawn in such that the first three blisters 303, 305 and 307 are received by the six-pocket index wheel 306. Indeed, the first blister 303 is open as result off the peeling off of the lid sheet 309 around the beak 310 at the opening station. Figure 4f thus, may correspond to a first dispensing position, although more often the first blister is not filled with medicament such that Figure 4f would correspond to a primed 'false start' position. Importantly, in the position shown in Figure 4f the leading end of the base sheet 309 and lid sheet 311 are received by the respective base sheet and lid sheet take-up spindles 316, 318. The spindle drive action thus, acts on the directly on the base sheet 309 and lid sheet 311 to enable the further driving of the strip 301 through the dispenser 300 for dispensing of medicament from the blisters 305, 307.

Figures 5a and 5b show alternative means of enabling access to the interior 523a, 523b of the housing 524a, 524b of a medicament dispenser cassette 500a, 500b to enable the receipt of leader strip(s) and medicament carrier(s) by the strip-receiving chamber 502a, 502b thereof.

In Figure 5a, the housing 524a of the cassette 500a is provided with a top-opening door 570a hinged at hinged point 572a. Once leader and medicament carrier strip (not shown) has been appropriately inserted the door 570a is dosed and typically sealed to provide a secure enclosure for the medicament carrier. In Figure 5b, the housing 524b of the cassette 500b is similarly provided with a side-opening door 570b hinged at hinge point 572b.

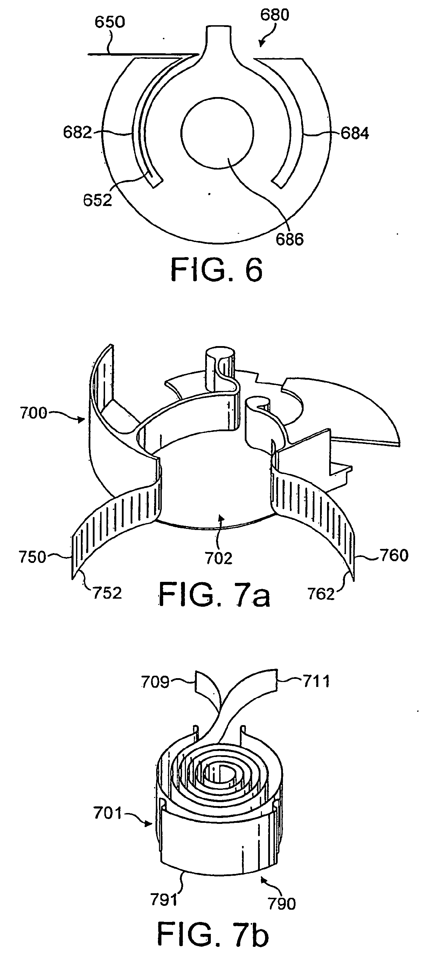

Figure 6 shows the form of a bobbin 680 suitable for attachment to the leading end 652 of a leader strip 650. The bobbin 680 may be seen to comprise circumferential slits 682, 684 for receipt of strip 650 and spindle mounting head 686, which is shaped for co-operation with, or in other aspects to replace, a spindle (e.g. a lid or base sheet take-up spindle 216, 218 as shown in Figure 2). The bobbin 680 is employed in aspects herein whereby in a pro-step, the leader strip 650 is associated with the bobbin 680 by receipt of its leading end 652 in slit 682 and the bobbin-headed leader strip assembly 650, 680 then introduced into a dispenser. In these aspects, it will be appreciated that the function is performed by the bobbin-headed leader strip assembly 650, 680 rather than just by a leader strip 650 alone.

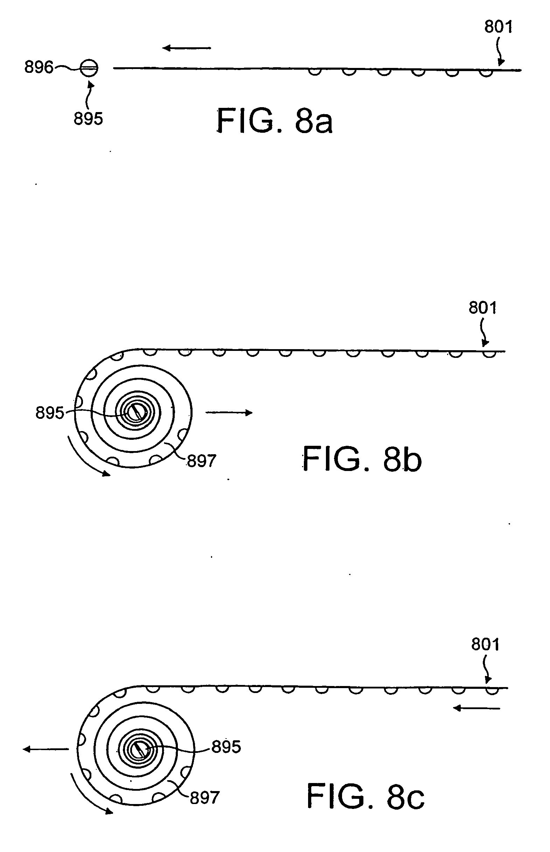

Figures 7a and 7b illustrate a development of the loading method herein in which a dispenser 700 which has been pre-loaded with leader strip 750, 760 (e.g. as shown in the steps illustrated in Figures 4a to 4c) is associated with a pre-coiled blister strip 701 medicament carrier comprised within a clip-form cassette 790. The pre-coiling of the blister strip 701 may be conducted by any suitable method of the invention including those shown in Figures 8a to 8c below. The base sheet 709 and lid sheet 711 of the blister strip 701 protrude from the clip-form cassette 790 and are arranged for easy fixing to the relevant ends 752, 762 of the leader strip 750, 760. As in previous examples, once so fixed the blister strip 701 may be readily drawn into the dispenser by the action of the leader strips 750, 760. The clip-form cassette 790 is arranged for snap-fit engagement with the dispenser 700 such that when engaged a wall 791 of the cassette forms part of the dispenser housing. It will also be appreciated that when so engaged, the blister strip 701 is generally received in the strip-receiving chamber 702 of the dispenser housing 700 from which it may be readily drawn in by action of the leaders 750, 760.

Figures 8a to 8c generally show blister strip 801 coiling methods. A pre-coiling step may be conducted prior to fixing of the blister strip to leader strip. Additionally, any pre-coiling may be followed by a step where the coiled blister strip is introduced into a cassette, such as the simple clip-form cassette 790 of Figures 7a and 7b.

In Figure 8a, the leading end of a blister strip 801 is received by a spindle 895 having a slit 896 provided therein. The spindle 895 is both rotatable and movable on a horizontal axis. In a first alternative shown in Figure 8b, the spindle 895 is rotated anti-clockwise whilst the strip 801 is kept generally static. A coiled strip 897 thereby results wherein the coil 897 is moving generally in a left to right direction, as shown. In a second alternatively in accordance with the present invention, shown in Figure 8c, the spindle 895 also rotates anti-clockwise but the strip 801 is moved in a right to left direction. Overall, the coiled strip 897 thereby moves in a right to left direction. Other coiling methods, which represent variations of these particular examples are also envisaged.

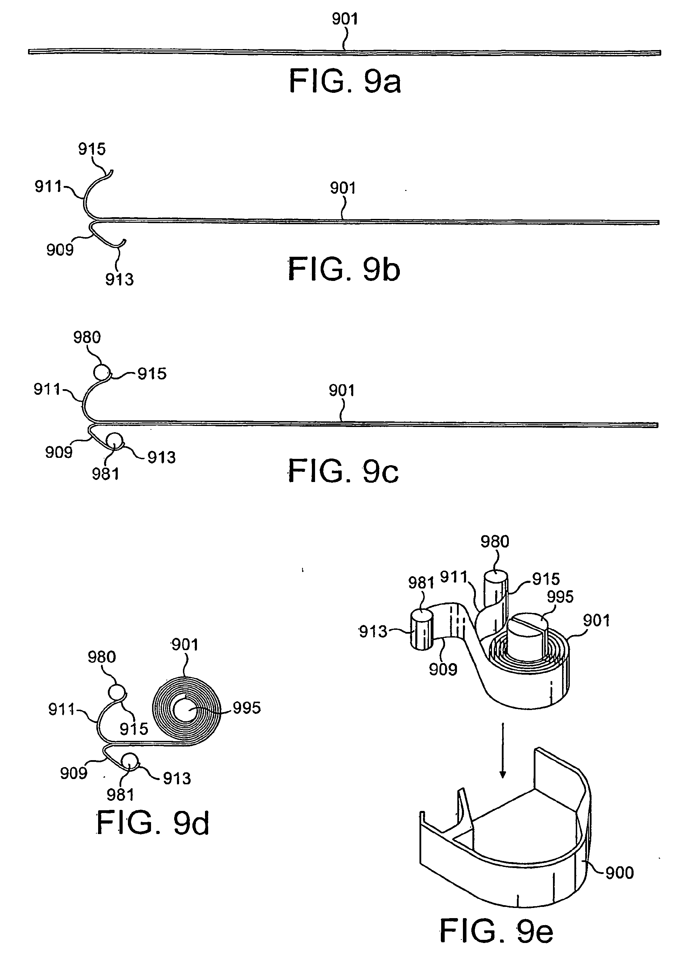

Figures 9a to 9e show the sequential steps involved in one method of pre-coiling an elongate strip form medicament carrier and loading it into the housing of a medicament dispenser.

In Figure 9a, an elongate form medicament carrier 901 is first cut to length. The medicament carrier 901 has the general flexible strip form of that carrier shown in Figure 1. Pockets for containing medicament are arranged in series along its length, although for simplicity of representation these are not shown in Figures 9a to 9a.

In Figure 9b, the leading ends 913, 916 of the base sheet 909 and lid sheet 911 of the medicament carrier strip are peeled apart. In Figure 9c, bobbins 980, 981 are respectively attached to those leading ends 913, 915. The medicament carrier strip 901 is then coiled around a spindle 995 as shown in Figure 9d, by a suitable coiling method in accordance with the present invention such as the dynamic coiling method of Figures 8a to 8c.

In Figure 9e, the pre-coiled strip 901 and bobbin 980, 981 assembly is loaded into a suitable medicament dispenser housing 900 by placement therein. It will be appreciated that within the housing 900 the bobbin-headed leading ends 913, 915 will then be associated with suitable base sheet and lid sheet anchors (e.g. as shown in Figure 2) for drivable peeling of the strip 901, in use.

It may be appreciated that any of the parts of the device or any medicament thereof which contacts medicament may be coated with materials such as fluoropolymer materials (e.g. PTFE or FEP) which reduce the tendency of medicament to adhere thereto. Any movable parts may also have coatings applied thereto which enhance their desired movement characteristics. Frictional coatings may therefore be applied to enhance frictional contact and lubricants (e.g. silicone oil) used to reduce frictional contact as necessary.

The device of the invention is suitable for dispensing medicament products particularly for the treatment of respiratory disorders such as asthma and chronic obstructive pulmonary disease (COPD), bronchitis and chest infections.

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α,9α-difluoro-11β-hydroxy-16α-methyl-3-oxo-17α-propionyloxy-androsta-1,4-diene-17β-carbothioic acid S-(2-oxo-tetrahydro-furan-3-yl) ester, antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g. as free acid or potassium salt), diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g.. as alkali metal or amine salts or as acid addition salts) or as esters (e.g., lower alkyl esters) or as solvates (e.g., hydrates) to optimise the activity and/or stability of the medicament

In one aspect, preferred medicaments are selected from albuterol, salmeterol, fluticasone propionate and bedomethasone dipropionate and salts or solvates thereof, e.g., the sulphate of albuterol and the xinafoate of salmeterol.

In one aspect, the medicament dispenser device herein is suitable for dispensing medicament combination products. Preferred components of combinations of active ingredients contain a bronchodilator in combination with an anti-inflammatory. The bronchodilator is suitably a beta-agonist, particularly a long-acting beta-agonist (LABA). Suitable bronchodilators include salbutamol (e.g., as the free base or the sulphate salt), salmeterol (e.g., as the xinafoate salt) and formoterol (eg as the fumarate salt). The anti-inflammatory is suitably an anti-inflammatory steroid. Suitably anti-inflammatory compounds include a beclomethasone ester (e.g., the dipropionate), a fluticasone ester (e.g., the propionate) or budesonide or any salt or solvate thereof. One preferred combination of components comprises fluticasone propionate and salmeterol, or any salt or solvate thereof (particularly the xinafoate salt). A further combination of components of particular interest is budesonide and formoterol or any salt or solvate thereof (e.g. formoterol as the fumarate salt).

Generally, powdered medicament particles suitable for delivery to the bronchial or alveolar region of the lung have an aerodynamic diameter of less than 10 micrometers, preferably less than 6 micrometers. Other sized particles may be used if delivery to other portions of the respiratory tract is desired, such as the nasal cavity, mouth or throat The medicament may be delivered as pure drug, but more appropriately, it is preferred that medicaments are delivered together with excipients (carriers) which are suitable for inhalation. Suitable excipients include organic excipients such as polysaccharides (i.e. starch, cellulose and the like), lactose, glucose, mannitol, amino acids, and maltodextrins, and inorganic excipients such as calcium carbonate or sodium chloride. Lactose is a preferred excipient

Particles of powdered medicament and/or excipient may be produced by conventional techniques, for example by micronisation, milling or sieving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well-known methods, such as by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 100:1 to 1:1 may be used. At very low ratios of exdpient to drug, however, the drug dose reproducibility may become more variable.

The dispenser device herein is in one aspect suitable for dispensing medicament for the treatment of respiratory disorders such as disorders of the lungs and bronchial tracts including asthma and chronic obstructive pulmonary disorder (COPD). In another aspect, the invention is suitable for dispensing medicament for the treatment of a condition requiring treatment by the systemic circulation of medicament, for example migraine, diabetes, pain relief e.g. inhaled morphine.

Accordingly, there is provided the use of a dispenser device herein for the treatment of a respiratory disorder, such as asthma and COPD. Alternatively, there is provided a method of treating a respiratory disorder such as, for example, asthma and COPD, which comprises administration by inhalation of an effective amount of medicament product as herein described from a device of the present invention.

It will be understood that the present disclosure is for the purpose of illustration only and the invention extends to modifications, variations and improvements thereto within the scope of the claims.

## Claims

1. A method of coiling a medicament carrier (801; 901) in preparation for loading the coiled carrier into a housing of a medicament dispenser, said carrier having the form of an elongate, blister pack strip and having multiple distinct medicament doses carried thereby, the method comprising:
(a) receiving the leading end of the strip by a spindle (895; 995); and
(b) forming a coil of the strip on the spindle by rotating said spindle whilst moving the strip in a lateral sense on a path towards the spindle,
wherein in step (b) the rotating spindle is moved in the same lateral sense as the strip.

2. A method of coiling according to claim 1, wherein the spindle frictionally engages the strip.

3. A method of coiling according to claim 1, wherein the end of the strip is received within a slit (896) provided to the spindle.

4. A method according to any one of claims 1 to 3, wherein the strip is in the form of a peelable blister strip (101) comprising a base sheet (109), in which blisters are formed to define pockets (103,105,107,109) therein for containing distinct medicament dose portions, and a lid sheet (111) which is hermetically sealed to the base sheet except in the region of the blisters in such a manner that the lid sheet and the base sheet can be peeled apart.

5. A method according to any one of the preceding claims comprising associating the coiled strip (897) with a retaining means for retaining the coiled form.

## Patentansprüche

1. Verfahren zum Aufrollen eines Medikamententrägers (801; 901) bei der Vorbereitung zum Laden des aufgerollten Trägers in ein Gehäuse eines Medikamentenspenders, wobei der Träger die Form eines länglichen Blisterverpackungsstreifens aufweist und durch diesen mehrere abgegrenzte Medikamentendosen getragen werden, wobei das Verfahren aufweist:
(a) Empfangen des Führungsendes des Streifens durch eine Spindel (895; 995); und
(b) Bilden einer Rolle aus dem Streifen auf der Spindel durch Rotieren der Spindel während des Bewegens des Streifens in einer seitlichen Richtung auf einem Pfad in Richtung der Spindel,
wobei die rotierende Spindel im Schritt (b) in der gleichen seitlichen Richtung wie der Streifen bewegt wird.

2. Verfahren zum Aufrollen nach Anspruch 1, bei dem die Spindel den Streifen über Reibung in Eingriff bringt.

3. Verfahren zum Aufrollen nach Anspruch 1, bei dem das Ende des Streifens in einem Schlitz (896) aufgenommen wird, der an der Spindel bereitgestellt ist.

4. Verfahren nach einem der Ansprüche 1 bis 3, bei dem der Streifen in Form eines abziehbaren Blisterstreifens (101) ist, der eine Basislage (109), in der Blister ausgebildet sind, um darin Taschen (103, 105, 107, 109) zum Aufnehmen abgegrenzter Medikamentendosierungsmengen zu definieren, und eine Decklage (111) aufweist, die mit Ausnahme des Bereichs der Blister hermetisch mit der Basislage auf eine Weise versiegelt ist, dass die Decklage und die Basislage voneinander gelöst werden können.

5. Verfahren nach einem der vorstehenden Ansprüche, mit Verknüpfen des aufgerollten Streifens (897) mit einem Haltemittel zum Halten der aufgerollten Form.

## Revendications

1. Procédé d'enroulement d'un support de médicament (801 ; 901), lors de la préparation pour charger le support enroulé dans un logement d'un distributeur de médicament, ledit support ayant la forme d'une bande d'emballage de blister allongée et permettant de transporter ainsi de multiples doses de médicament distinctes, le procédé comprenant :
(a) la réception de l'extrémité avant de la bande par une broche (895, 995), et
(b) la formation d'une bobine de la bande sur la broche, en faisant tourner ladite broche tout en déplaçant la bande dans un sens latéral sur un parcours vers la broche,
dans lequel, à l'étape (b), la broche rotative est déplacée dans le même sens latéral que la bande.

2. Procédé d'enroulement selon la revendication 1, dans lequel la broche vient en prise avec la bande par frottement.

3. Procédé d'enroulement selon la revendication 1, dans lequel l'extrémité de la bande est reçue dans une fente (896) ménagée sur la broche.

4. Procédé selon l'une quelconque des revendications 1 à 3, dans lequel la bande est sous la forme d'une bande de blister pelable (101) comprenant une feuille de base (109), dans laquelle des blisters sont formés pour définir des poches (103, 105, 107, 109), à l'intérieur, afin de contenir des portions de dose de médicament distinctes et une feuille de couverture (111) qui est hermétiquement scellée à la feuille de base, excepté dans la zone des blisters de sorte que la feuille de couverture et la feuille de base puissent se décoller l'une de l'autre.

5. Procédé selon l'une quelconque des revendications précédentes, comprenant l'association de la bande enroulée (897) à un système de retenue permettant de retenir la forme enroulée.
